Europäisches Patentamt

**European Patent Office**

**Office européen des brevets**

(11) Numéro de publication: **0 229 573 B1**

## (12) FASCICULE DE BREVET EUROPEEN

(45) Date de publication de fascicule du brevet: **21.07.93** (51) Int. Cl.5: **G01N 17/00**

(21) Numéro de dépôt: **86402960.8**

(22) Date de dépôt: **30.12.86**

(54) **Procédé et dispositif d'analyse de l'effet corrosif du sol et de son environnement sur une structure enterrée et application à la localisation de cet effet.**

(30) Priorité: **31.12.85 FR 8519501**

(43) Date de publication de la demande:
**22.07.87 Bulletin 87/30**

(45) Mention de la délivrance du brevet:
**21.07.93 Bulletin 93/29**

(84) Etats contractants désignés:
**AT BE DE GB IT NL**

(56) Documents cités:
**CA-A- 1 191 899      DE-A- 2 707 265**
**GB-A- 1 342 949      GB-A- 2 025 056**
**GB-A- 2 049 943      US-A- 4 611 175**

(73) Titulaire: **TOTAL RAFFINAGE DISTRIBUTION S.A.**
**84, rue de Villiers**
**F-92300 Levallois Perret(FR)**

(72) Inventeur: **Saumade, Frank**
**27, rue Michelet**
**F-78310 Plaisir(FR)**
Inventeur: **Fontaine, Guy**
**2, Impasse des Carrières**
**F-75016 Paris(FR)**

(74) Mandataire: **Jolly, Jean-Pierre et al**
**Cabinet Jolly 54, rue de Clichy**
**F-75009 Paris (FR)**

Rank Xerox (UK) Business Services
(3. 10 / 3.6 / 3.3. 1)

**Description**

La présente invention concerne un procédé d'analyse de l'effet corrosif du sol et de son environnement sur une structure métallique enterrée et son application à la localisation de cet effet. L'invention concerne également un dispositif de mise en oeuvre de ce procédé et de cette application.

Par structure métallique enterrée, on entend toute infrastructure métallique enterrée du type canalisations et citernes de station service, cuves à fioul domestique, bacs de stockage et tous les réseaux de transport ou de distribution de fluides organiques et minéraux, sous forme liquide ou gazeuse.

Il est connu que les structures métalliques enterrées se corrodent par formation de piles électriques à leur surface, la corrosion se développant dans les zones anodiques de ces piles. La création de ces piles dépend d'au moins deux facteurs, qui sont l'hétérogénéité des sols traversés par la structure et la composition métallique de celle-ci. Par ailleurs, la présence de courants vagabonds provenant de sources extérieures situées dans l'environnement de la structure est aussi génératrice de corrosion.

La composition métallique de la structure est contrôlable, dans la mesure où l'on connaît généralement le matériau composant la structure avant de l'enterrer, mais l'hétérogénéité du sol et la présence de courants vagabonds ne le sont pas. En particulier, il est difficile de connaître, de la surface du sol, la qualité du sol que traverse la structure, millimètre par millimètre. Il suffit qu'un caillou, une cale de bois ou de l'humus rencontre la surface de la structure, par ailleurs déjà en contact avec un matériau différent de par sa nature, son acidité, sa densité, sa granulométrie ou son humidité, pour qu'apparaisse un effet de pile au niveau de la cale, du caillou ou des déchets humiques, la pile se créant généralement à la limite des deux matériaux différents, à la surface de la structure.

Pour essayer de détecter, depuis la surface du sol, l'effet corrosif de ce dernier et de l'environnement, vis-à-vis d'une structure enterrée, il est d'usage d'effectuer ponctuellement la mesure de trois caractéristiques à l'aplomb de la structure examinée, à savoir:

- la différence de potentiel entre la surface du sol et la structure enterrée, mesurée par un voltmètre entre une électrode référencée, du type Cu/CuSO$_4$, dont la partie sensible est mise en contact avec la surface du sol humidifiée, et par exemple une vanne reliée à la structure enterrée et émergeant de la surface du sol,
- la résistivité du sol, mesurée par la méthode dite du "pont de Wenner", selon une technique connue,
- et le pH mesuré, par carottage du sol, dans le voisinage de la structure enterrée.

La comparaison des valeurs moyennes de ces mesures, pratiquées à des intervalles plus ou moins réguliers de un mètre au minimum, pour ces trois paramètres, et des valeurs généralement retenues comme significatives d'un effet corrosif important du sol ou de son environnement permet de décider s'il est nécessaire ou non d'appliquer sur la structure une protection cathodique.

Cette procédure de détection de l'effet corrosif du sol et de son environnement permet de savoir si ceux-ci sont plutôt corrosifs ou non, mais non pas de localiser exactement les zones anodiques où se développe la corrosion, ni de déterminer les dimensions de ces zones. En outre, quelle que soit la décision prise au regard des résultats obtenus par la mise en oeuvre de cette procédure, les conséquences en sont ou deviennent très coûteuses : on est toujours obligé,à court ou moyen terme, de prévoir la mise en place d'une protection cathodique, ou de déterrer la structure pour rechercher les "trous" résultant de la corrosion, qui sont difficiles, voire impossibles, à détecter de la surface du sol par ce processus.

US-A-4 611 175 décrit un procédé et un dispositif pour déterminer l'état de corrosion d'un objet métallique enterré. Le dispositif comprend une électrode enfoncée dans le sol, une source de courant continu connectée à l'objet métallique enterré et à l'électrode en vue de charger électriquement cet objet et un moyen de mesure de l'affaiblissement de cette charge, apte à fournir une indication de l'état de corrosion de cet objet.

Le but de la présente invention est de disposer d'un procédé fiable de détection de la corrosion, permettant de localiser avec précision les zones corrodées ou favorables à la corrosion et leur étendue sur la structure enterrée, et de quantifier l'effet corrosif du sol ou de son environnement vis-à-vis de la structure.

A cet effet, contrairement à la procédure employée dans la technique connue, on propose de suivre, par des mesures en continu ou "semi-continues" entre la surface du sol et la structure enterrée, l'évolution quasi-continue de paramètres tels que les différences de potentiel entre la structure enterrée et la surface du sol, et la résistivité du sol au voisinage de la structure. En effet, l'interprétation de ces évolutions permet de déterminer le sens et l'intensité du courant, au niveau de la pile formée à la surface de la structure, le courant sortant traduisant la présence d'une zone anodique favorable à la corrosion.

La présente invention a par conséquent pour objet un procédé d'analyse en continu ou en discontinu de l'effet corrosif du sol et de son environnement sur une structure métallique enterrée, caractérisé par les

2

opérations suivantes :

A) la mesure d'au moins deux différences de potentiel à l'aide d'au moins une électrode de potentiel de référence connu placée en contact avec la surface du sol, ledit sol constituant l'électrolyte et la structure enterrée une électrode métallique, ces différences de potentiel étant les suivantes :

- une différence de potentiel V1, entre la surface du sol et la structure enterrée,
- une différence de potentiel V2, entre la surface du sol et la structure enterrée, lorsque le potentiel de ladite structure est ramené au moyen d'une liaison électrique à une électrode métallique reposant sur le sol,
- et au moins une troisième différence de potentiel V3, entre la surface du sol et la structure enterrée, se décomposant comme V1, mais obtenue en modifiant la différence de potentiel qui est engendrée par l'action d'échange électrique existant entre ladite structure et le sol ;

B) la visualisation et l'enregistrement par un organe de visualisation et d'acquisition de données de ces différences de potentiel ;

C) le traitement de ces données à l'aide de moyens informatiques pour calculer la différence (V1 - V2), éventuellement au moins une différence (V3 - V1) ;

D) la détermination à partir de ces différences de potentiel de l'effet corrosif du sol et de l'environnement sur la structure enterrée.

Pour mesurer la différence de potentiel V1, on peut disposer l'électrode de potentiel de référence connu, de préférence une électrode $Cu/CuSO_4$, à la verticale du point concerné de ladite structure enterrée.

Pour mesurer la différence de potentiel V2, on peut choisir d'accoler l'électrode métallique ramenant le potentiel de la structure au sol à l'électrode de potentiel de référence connu, par exemple $Cu/CuSO_4$ et de les placer sur le sol à une distance $d_2$ au sol de la verticale du point concerné de ladite structure, $d_2$ étant comprise de préférence entre 0 et 50cm. Cette disposition respective des deux électrodes a pour avantage d'éliminer ou de réduire au minimum l'effet lié à l'échange électrique entre le sol et la structure.

En vue de mesurer la différence de potentiel V3, selon l'invention, il est possible de modifier la différence de potentiel d'échange électrique sol-structure enterrée en agissant sur l'un des deux paramètres constitutifs de cette différence de potentiel, c'est-à-dire sur l'intensité du courant d'échange entre la structure et le sol ou sur la résistance du sol entre la surface du sol et la structure enterrée.

Selon un mode de mise en oeuvre préféré de ce procédé, pour modifier l'intensité du courant d'échange entre la structure et le sol, la Demanderesse propose de générer au moins un courant électrique entre l'électrode de potentiel de référence telle que $Cu/CuSO_4$ et l'électrode constituée par la structure enterrée.

Pour modifier la résistance du sol, il est possible de déplacer sur le sol l'électrode $Cu/CuSO_4$ d'une distance $d_3$ de la verticale du point analysé de la structure, $d_3$ étant comprise de préférence entre 50cm et 200cm, et de mesurer V3 comme V1 entre l'électrode $Cu/CuSO_4$ et l'électrode "structure enterrée".

La présente invention a également pour objet un dispositif de mise en oeuvre du procédé caractérisé en ce qu'il comprend :

- un système de mesure constitué d'un capteur comprenant au moins une électrode $Cu/CuSO_4$ à laquelle est accolée une électrode métallique contenant du fer, et d'au moins un voltmètre,
- au moins un système de visualisation et d'acquisition de données constitué d'au moins un amplificateur, d'au moins un convertisseur analogique numérique, d'au moins un enregistreur magnétique et d'au moins un appareil de visualisation pour la lecture sur site de ces données, appartenant au groupe constitué par les afficheurs numériques et les enregistreurs électromécaniques,
- et d'un système de traitement de ces données constitué de moyens de calcul informatiques.

Le système de mesure de ce dispositif pourra également comprendre un générateur de courant.

Deux formes de réalisation des capteurs constituant une partie de ce système de mesure sont considérées comme préférées. Ce sont, d'une part, les capteurs à développement linéaire, en contact permanent avec le sol, assurant une mesure continue des différences de potentiel V1, V2 et éventuellement au moins V3, et d'autre part, les capteurs à développement ponctuel, décollés du sol entre chaque mesure et ne donnant que des mesures discontinues de ces différences de potentiel.

Les capteurs à développement linéaire selon l'invention comprennent au moins deux électrodes $Cu/CuSO_4$ en forme de roue creuse, X1 et X2, dont la périphérie constitue la partie sensible de l'électrode et est réalisée en au moins un matériau choisi dans le groupe constitué par le liège, le bois et plus généralement toute matière poreuse inerte électriquement. Les parois latérales en forme de disque de cette roue sont en une matière électriquement inerte, non poreuse, qui contient un disque de cuivre de diamètre inférieur à celui de ladite roue, et une solution saturée de sulfate de cuivre, $CuSO_4$. Les électrodes X1 et X2 sont disposées en parallèle et maintenues solidaires l'une de l'autre, X2 à une distance $d_2$ de X1. Le capteur comprend aussi au moins une troisième électrode $Cu/CuSO_4$, X3, placée à une distance $d_3$ de X1.

3

Le capteur comprend enfin au moins une électrode métallique, de préférence en acier doux, composée d'au moins un disque métallique, accolée à une des électrodes Cu/CuSO₄, X1, X2 ou X3.

Il est clair, pour l'Homme de l'Art, que ce type de capteur à développement linéaire peut comprendre plus de trois électrodes Cu/CuSO₄ et plus d'une électrode métallique, permettant de mesurer d'autres différences de potentiel entre la surface du sol et la structure.

Les capteurs à développement ponctuel, selon l'invention, peuvent comprendre, d'une part, au moins une électrode Cu/CuSO₄ obtenue à partir d'un cylindre en une matière inerte électriquement, non poreuse, bouché à sa base par un bouchon de matière poreuse choisi dans le groupe constitué par le bois, le liège ou plus généralement toute autre matière poreuse inerte électriquement, ledit cylindre contenant une solution saturée de sulfate de cuivre, CuSO₄, dans laquelle plonge une tige de cuivre assurant la liaison électrique de ladite électrode, et d'autre part, une électrode métallique enveloppant l'électrode Cu/CuSO₄ d'une gaine métallique, de préférence en acier doux. Pour mesurer les différents potentiels et, en particulier, V1 et V3, on peut soulever le capteur à développement ponctuel, s'il est unique, pour le déplacer, ce capteur ne donnant alors que des mesures discontinues.

Il est plus avantageux d'utiliser un capteur à développement linéaire, lorsque le sol est plat et sans embûche, car il nécessite moins d'efforts physiques pour l'opérateur qui fait l'analyse et, surtout, il donne une vision de l'évolution des différences de potentiel V1, V2, et, éventuellement, au moins V3, en continu le long de la structure, sans omettre un élément de surface de celle-ci.

Néanmoins, lorsque la topographie du sol est plus accidentée, par exemple lorsqu'un conduit de distribution ou de transport traverse le sol d'une forêt, les capteurs à développement linéaire roulent difficilement à cause des obstacles rencontrés. Dans ce cas, l'utilisation du capteur à développement ponctuel est plus pratique.

Le système de visualisation et d'acquisition des données est choisi de préférence portable, pour être transporté sur le site et être relié en permanence avec le capteur, afin de suivre l'évolution des différences de potentiel V1, V2 et éventuellement au moins V3.

Par contre, les moyens de calcul informatiques constituant l'organe de traitement de données peuvent être transportables, comme la plupart des micro-ordinateurs, ou non transportables, comme certains ordinateurs centraux.

Ce système de traitement de données selon l'invention a pour fonction de relire les données V1, V2 et, éventuellement, au moins V3, enregistrées sur le site par le système de visualisation et d'acquisition de données, de normaliser les valeurs de V1, V2 et V3, en fonction des potentiels réels de la ou des électrodes Cu/CuSO₄ utilisées qui sont connus, et de calculer la différence (V1 - V2), la différence (V1 - V3), et toutes autres différences de différence de potentiel par rapport à V1.

Ces différences sont utilisées pour localiser et quantifier, du moins en relatif, l'intensité de l'effet corrosif du sol et de l'environnement vis-à-vis de la structure enterrée.

La présente invention a également pour objet l'application de ce procédé et de ce dispositif d'analyse de l'effet corrosif du sol et de son environnement vis-à-vis d'une structure métallique enterrée à la localisation sur le site des zones anodiques favorables à la corrosion, application caractérisée par les opérations suivantes:

- déplacer de façon continue ou discontinue le ou les capteurs du système de mesure le long de la structure enterrée sur le sol préalablement humidifé pour assurer les contacts capteurs-sol,
- lire sur l'appareil de visualisation du système de mesure les valeurs de V1 et de V2,
- localiser et signaler les zones anodiques, lorsque V1 est supérieur en valeur absolue à la valeur absolue de V2.

D'autres caractéristiques et avantages de l'invention apparaîtront dans la description détaillée qui va suivre de certaines formes de mise en oeuvre, données à titre d'exemple non limitatif. Dans cette description, on se référera aux dessins schématiques annexés, sur lesquels:

La figure 1 représente un organe de mesure comprenant un capteur à développement ponctuel, positionné au-dessus d'une conduite métallique enterrée;

Les figures 2A et 2B sont respectivement une vue de côté d'une électrode Cu/CuSO₄ circulaire, à laquelle est accolée l'électrode métallique, et une coupe suivant la ligne X-X de la figure 2A;

La figure 3 représente un capteur à développement linéaire comprenant trois électrodes circulaires, X1, X2 et X3, et une électrode métallique;

Les figures 4A et 4B représentent deux capteurs linéaires permettant d'effectuer des mesures en continu de V1, V2 et V3, suivant que la résistance du sol est modifiée (Figure 4A) ou que l'intensité du courant est modifiée par génération d'un courant alternatif (Figure 4B);

La figure 5 est un diagramme des fonctions du système de mesure associé au système de visualisation et d'acquisition de données;

4

La figure 6 est un diagramme des fonctions du système de traitement selon l'invention.

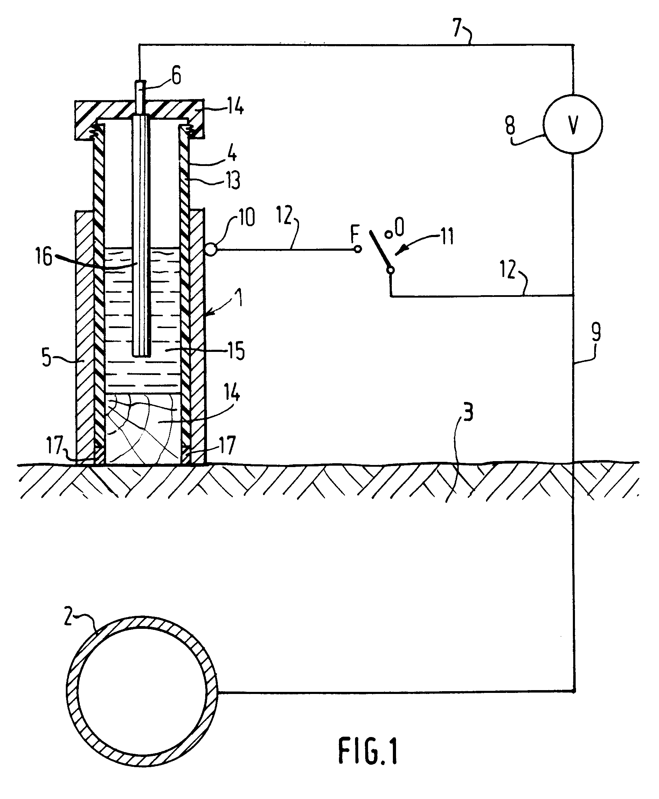

On se réfèrera d'abord à la figure 1. Le capteur à développement ponctuel 1 est positionné à la verticale de la conduite métallique enterrée 2, sur le sol 3.

Ce capteur 1 est constitué d'une électrode 4, Cu/CuSO₄, de forme cylindrique, et d'une électrode métallique 5 en acier, qui enveloppe l'électrode 4 d'une gaine d'épaisseur égale à 4 mm, jusqu'aux trois quarts de sa hauteur. Le capteur 1 est relié via le contact 6 de l'électrode 4 et la liaison électrique 7 au voltmètre 8, lui-même relié par la liaison électrique 9 à la conduite 2 par l'intermédiaire d'une vanne non représentée sur la figure, affleurant à la surface du sol 3. Le capteur 1 est également relié à la conduite 2 via le contact 10 de l'électrode 5, relié à l'interrupteur 11 par la liaison électrique 12, qui rejoint la liaison électrique 9 entre le voltmètre 8 et la vanne.

La paroi cylindrique 13 de l'électrode Cu/CuSO₄ est réalisée en une matière inerte électriquement, par exemple du polychlorure de vinyle. Elle est bouchée à son extrémité supérieure par un bouchon isolé 14, de nature comparable à celle de la paroi cylindrique 13, et à son extrémité inférieure, partie sensible de l'électrode 4, par un bouchon de bois 14, dépassant légèrement du cylindre 13. Cette électrode contient, en outre, une solution saturée 15 de sulfate de cuivre CuSO₄, dans laquelle plonge une tige de cuivre 16, qui traverse le bouchon 14 et constitue à son extrémité supérieure le contact 6 du capteur 1.

L'enveloppe métallique constituant l'électrode 5 déborde de la paroi cylindrique 13 de l'électrode 4 de la même hauteur que le bouchon 14, la cavité circulaire 17 entre le bouchon 14 et l'électrode 5 étant remplie d'une substance inerte électriquement, par exemple de l'araldite.

Pour mesure V1 ou V2, le capteur 1 est positionné au-dessus de la conduite 2 sur le sol 3 humidifié. Le voltmètre 8 indique la différence de potentiel V1, lorsque l'interrupteur 11 est en position "ouvert" (O), et la différence de potentiel V2, lorsque l'interrupteur 11 est en position "fermé" (F). Pour mesurer V3, le capteur 1 est déplacé d'une distance $d_3$ sur le sol, de façon à ne plus être à la verticale de la conduite 2, et l'interrupteur est ouvert (O).

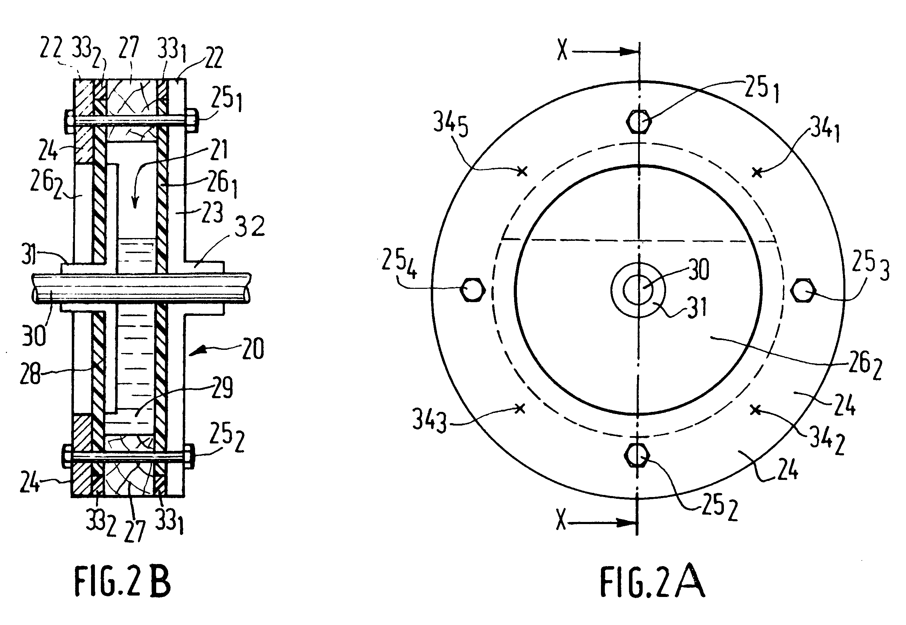

Dans le capteur 20 des figures 2A et 2B, l'électrode Cu/CuSO₄,21, est circulaire, en forme de roue de diamètre égal à 318 mm, et elle est accolée à une électrode métallique en acier doux 22, composée d'un disque en acier doux 23 de même diamètre que la roue constituant l'électrode 21 et d'épaisseur égale à 5 mm, et d'un anneau 24 plat de même diamètre extérieur et de même épaisseur que le disque 23, dont le diamètre intérieur est égal à 200 mm. Quatre boulons acier $25_1$, $25_2$, $25_3$, $25_4$, jouent le rôle de contact entre le disque 23 et l'anneau 24.

L'électrode Cu/CuSO₄ 21 comporte deux parois circulaires parallèles $26_1$ et $26_2$ de diamètre égal à 310 mm et d'épaisseur égale à 3 mm en une matière inerte électriquement, ici du TECHNYL (Marque déposée). Ces parois $26_1$ et $26_2$ sont maintenues parallèles par une couche de bois de largeur égale à 25 mm et d'épaisseur égale à 25 mm, disposée à la périphérie de ces parois, qui constitue la partie sensible 27 de l'électrode Cu/CuSO₄, constamment en contact avec le sol. Cette partie sensible 27 déborde d'une hauteur égale à 4 mm des parois $26_1$ et $26_2$.

A l'intérieur de la roue constituant l'électrode Cu/CuSO₄ 21, un disque de cuivre 28, de diamètre égal au diamètre intérieur de l'anneau 24, est disposé au contact de la paroi $26_2$ et plonge dans une solution saturée 29 de sulfate de cuivre, CuSO₄, qui remplit la roue aux deux tiers.

Les deux électrodes sont traversées en leur centre par un axe isolé électriquement 30, par exemple en TECHNYL, de diamètre égal à 20 mm et de longueur égale à 100 mm, cet axe jouant le rôle d'axe de rotation du capteur 20.

La paroi $26_2$ est percée d'un trou de diamètre supérieur au diamètre de l'axe 30, afin de laisser le passage à un cylindre de cuivre 31, dont le diamètre intérieur est égal à celui dudit axe 30 et qui est raccordé au disque 28, en assurant le contact électrique de l'électrode Cu/CuSO₄ 21 à l'extérieur de celle-ci.

Le disque 23 de l'électrode 22, accolé à la paroi $26_1$ de l'électrode Cu/CuSO₄ 21, est percé d'un trou de même diamètre que celui de l'axe 30, comme la paroi $26_1$. A ce niveau, le contact électrique de l'électrode 23 est assuré par un cylindre métallique 32 raccordé au disque 23, de diamètre interne égal au diamètre de l'axe isolé 30. Les rainures $33_1$ et $33_2$ sont remplies d'araldite.

Sur la figure 2A, le capteur 20 est vu du côté de la paroi $26_2$ de l'électrode de cuivre CuSO₄ 21. On voit l'anneau 24 percé de huit trous remplis par quatre boulons en acier doux $25_1$, $25_2$, $25_3$, $25_4$ et quatre boulons en nylon, $34_1$, $34_2$, $34_3$ et $34_4$. La paroi $26_1$, percée au centre d'un trou permet le passage du cylindre 31 en cuivre de contact de l'électrode Cu/CuSO₄ 21 et de l'axe isolé 30.

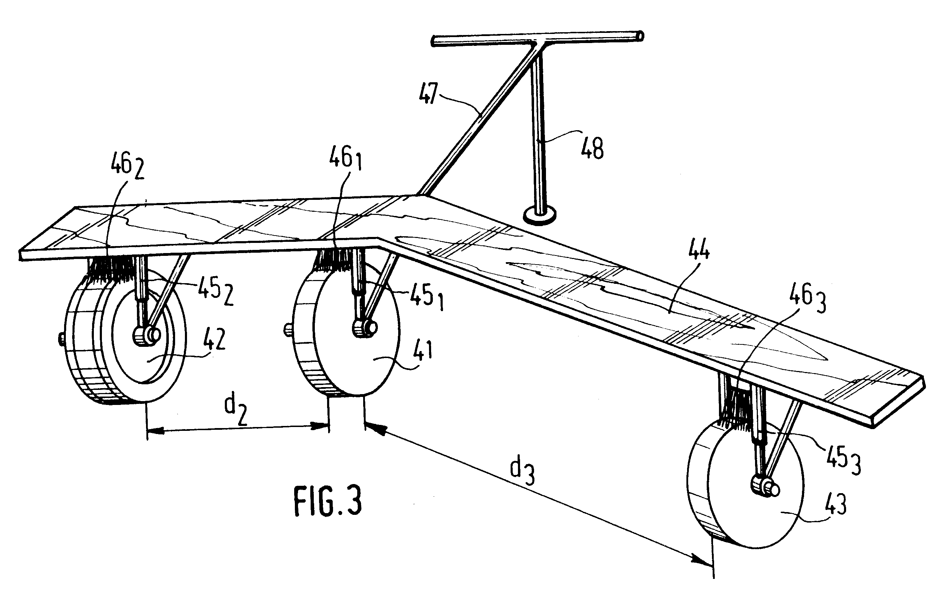

Le capteur à développement linéaire de la figure 3 est constitué de trois roues 41, 42 et 43 correspondant respectivement aux électrodes $X_1$, $X_2$ et $X_3$, $X_2$ comportant une électrode métallique accolée à l'électrode Cu/CuSO₄, est trois électrodes étant maintenues parallèles par l'intermédiaire d'un châssis 44, constitué de deux planches de bois raccordées aux axes des roues par des barres. Dans ce capteur, $d_2$ est

égal à 30 cm et $d_3$ est égal à 1 m.

Des amortisseurs doubles, $45_1$, $45_2$ et $45_3$, sont disposés au niveau des trois roues 41, 42 et 43, afin d'amortir les petites aspérités du sol.

De manière à ce que la partie sensible des électrodes $Cu/CuSO_4$ et de l'électrode métallique soit toujours propre, sans déchets ni cailloux, de petits balais, $46_1$, $46_2$ et $46_3$, sont placés sous les planches du châssis 44 de façon à balayer ces parties sensibles, lorsque le capteur roule.

Le capteur comporte en outre une poignée 47, en forme de T, raccordée au châssis, permettant de diriger ledit capteur. Sur cette poignée est soudée une béquille 48 pour maintenir le capteur linéaire en position de repos.

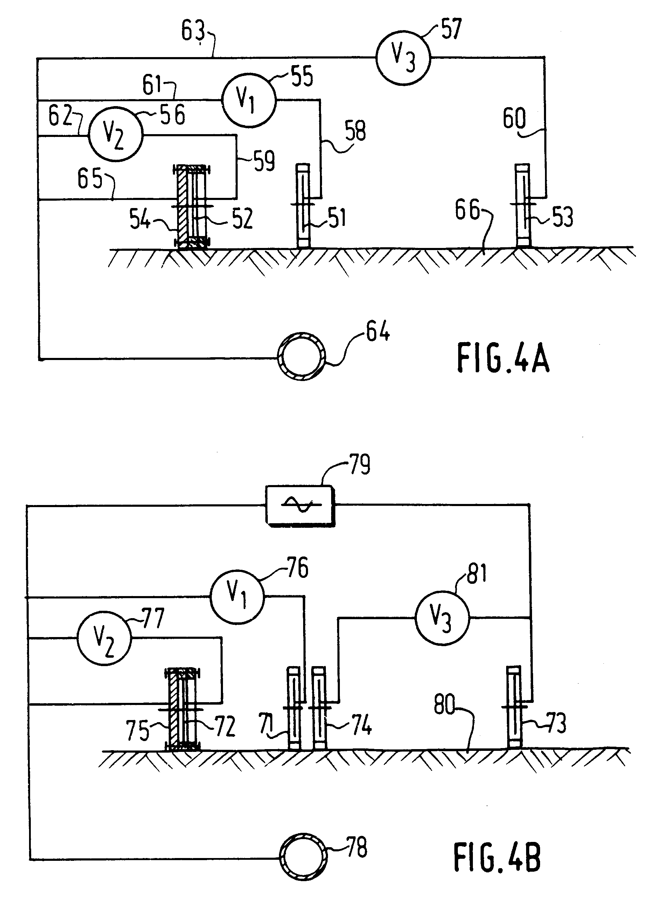

Le capteur linéaire de la figure 4A ne se compose que de trois électrodes $Cu/CuSO_4$, 51, 52 et 53, et d'une électrode métallique 54 accolée à l'électrode 52, toutes disposées sur le sol 66. Ce capteur est raccordé à trois voltmètres 55, 56 et 57, respectivement via les liaisons électriques 58, 59 et 60 issues des contacts électriques du cuivre des électrodes 51, 52 53.

La seconde borne des voltmètres 55, 56, 57 est reliée par les liaisons électriques 61, 62 et 63 au conduit enterré 64, disposé à la verticale sous l'électrode 51, via une vanne effleurant à la surface du sol, non représentée sur la figure. La liaison électrique 65 raccordant l'électrode métallique 54 à la vanne permet de ramener le potentiel du conduit 64 au niveau du sol, à proximité de l'électrode $Cu/CuSO_4$ 52.

Ce capteur permet de mesurer en continu les trois différences de potentiel, V1, entre le conduit 64 et l'électrode 52, V2, entre les deux électrodes 54 et 52, et V3, entre l'électrode 53 et le conduit 64.

Le capteur linéaire de la figure 4B, est constitué de quatre électrodes $Cu/CuSO_4$, 71, 72, 73 et 74, et d'une électrode métallique 75 accolée à l'électrode 72, toutes disposées sur le sol 80. Le voltmétre 76, relié à l'électrode 71 et à une vanne raccordée au conduit 78, et le voltmètre 77, relié à l'électrode 72 et à l'électrode 75, permettent, comme dans la figure précédente, de mesurer V1 et V2.

Pour mesurer V3, on a choisi d'engendrer un courant alternatif. Aussi, sur ce capteur, on a connecté l'électrode 73 à un générateur de courant alternatif, lui-même connecté à la conduite 78, via la vanne non représentée sur la figure.V3 est mesuré par un voltmètre relié à l'électrode 73 et à l'électrode 74 et disposé à une distance égale à 600 mm de l'électrode 71.

Comme avec le précédent capteur, V1, V2 et V3 peuvent être mesurés en continu lorsque le capteur roule sur le sol au-dessus de la conduite enterrée.

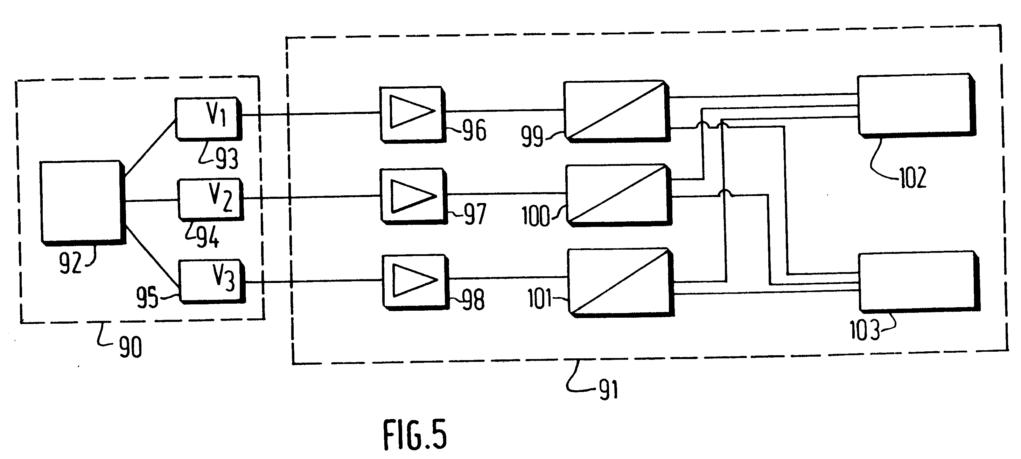

Sur la figure 5, le bloc 90 figure le système de mesure constitué par le capteur 92 et au moins les trois voltmètres 93, 94 et 95. Ces trois voltmètres sont connectés respectivement à trois amplificateurs 96, 97 et 98 du système de visualisation et d'acquisition de données 91, reliés eux-mêmes à des convertisseurs analogiques-numériques 99, 100, 101. Ces convertisseurs sont connectés à un appareil de visualisation 102, constitué d'afficheurs numériques pour lire sur le site les valeurs V1, V2, et V3, lorsqu'on roule ou déplace le capteur, et à un enregisteur magnétique 103, permettant la conservation de ces données.

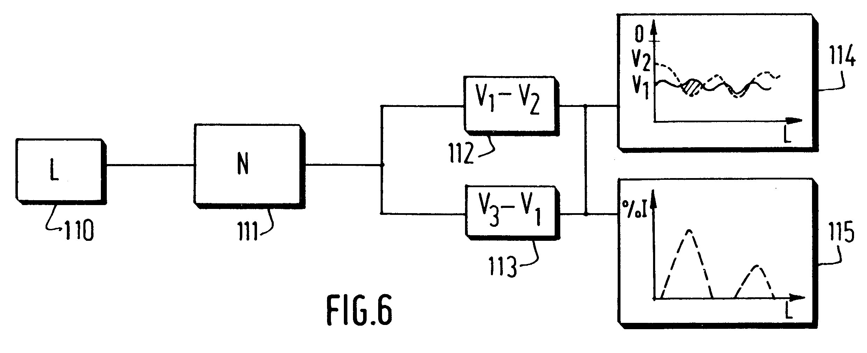

Le schéma de la Figure 6 illustre les fonctions du système de traitement de données, à savoir la lecture en 110 des données enregistrées, la normalisation en 111 des valeurs V1, V2, et V3, en fonction des valeurs des potentiels réels des électrodes $Cu/CuSO_4$ utilisées, qui sont mesurées hors site, et le calcul en 112 et 113 des différences V1 - V2 et V3 - V1.

Les valeurs obtenues pour ces différences permettent l'établissement de courbes 114 et 115 caractérisant respectivement les zones anodiques de corrosion en fonction de la longueur du conduit enterré et l'intensité relative de l'effet corrosif relatif du sol et de l'environnement du conduit enterré dans chacune de ces zones.

L'exemple ci-après est destiné à mieux illustrer la présente invention, sans pour autant en limiter la portée.

EXEMPLE

Deux diagnostics de corrosion ont été effectués pour une même station-service TOTAL d'un relais d'autoroute. Cette station possède six cuves de 60 $m^3$ et un collecteur permettant la distribution des fluides, qui constituent les structures métalliques enterrées.

Le premier diagnostic est un diagnostic témoin : il a été fait selon la procédure connue de l'homme de l'art, qui consiste à mesurer :
- la différence de potentiel V entre le sol et la structure, à l'aide d'une électrode $Cu/CuSO_4$, raccordée à un voltmètre, lui-même connecté à la structure;
- la résistivité $\rho$ sol au voisinage de la structure, à l'aide d'un pont de Wenner;
- le pH, à l'aide d'un pH mètre, par carottage du sol au voisinage de la structure.

Six points de mesures ont été traités. Les valeurs moyennes obtenues pour les mesures de V, $\rho$ et pH sont les suivantes:

- V = 480 mV,
- $\rho$ = 2000 $\Omega$ /cm²/cm,
- pH = 6,2.

Ces résultats traduisent une situation moyenne du sol au voisinage de la structure correspondant à une corrosion dite "active". Cette corrosion nécessite généralement la mise en place d'une protection cathodique sur toutes les structures.

Sur l'ensemble de la structure, le deuxième diagnostic a été obtenu à l'aide du procédé et du dispositif selon l'invention.

Pour mesurer $V_1$, $V_2$ et $V_3$, on a utilisé un capteur à développement linéaire à trois électrodes Cu/CuSO$_4$ tel que décrit en relation avec les figures 3 et 4A.

L'analyse des informations relatives à $V_1$, $V_2$ et $V_3$, mesurées sur le site, c'est-à-dire dans la station-service, a permis de localiser et de dimensionner cinq zones de corrosion, pour lesquelles $V_2$ est inférieur à $V_1$ en valeur absolue, ces cinq zones ayant été signalées ultérieurement, sur le plan de la station, par des couleurs différentes, traduisant des intensités différentes de l'effet corrosif du sol et de l'environnement vis-à-vis de la structure enterrée.

Les résultats obtenus par le procédé selon l'invention ont été rassemblés dans le tableau ci-après:

| Intensité relative de l'effet corrosif en % | | Blanc | Vert | Orange | Rouge foncé |
|---|---|---|---|---|---|
| zone | Longueur (cm) | 0 à 25 | 25 à 50 | 50 à 75 | 75 à 100 |
| 1 | 80 | X | | | |
| 2 | 160 | | X | | |
| 3 | 10 | | | | X |
| 4 | 80 | | | X | |
| 5 | 10 | X | | | |

Une intensité de l'effet corrosif égale à 100% correspond à une corrosion provoquant un début de fuite du fluide stocké dans la structure au niveau de la zone anodique. On constate qu'une seule zone, la zone 3, est très corrodée, tandis que les zones 2 et 4 sont moyennement corrodées et les zones 1 et 5 sont faiblement corrodées. Il sera donc possible, au vu de ces résultats, d'intervenir au niveau de zones corrodées des structures enterrées dans le sol de la station.

Contrairement au bilan du premier diagnostic témoin, le diagnostic de corrosion obtenu selon le procédé de l'invention permet de localiser les zones anodiques avec une précision de quelques centimètres et de quantifier les risques de corrosion et de fuites du produit à leur niveau.

**Revendications**

1. Procédé d'analyse en continu ou en discontinu de l'effet corrosif du sol et de l'environnement sur une structure métallique enterrée (2), caractérisé par les opérations suivantes:

   A.- la mesure d'au moins deux différences de potentiel à l'aide d'au moins une électrode (4) de potentiel de référence connu placée en contact avec la surface du sol, ledit sol constituant l'électrolyte et la structure enterrée (4) une électrode métallique, ces différences de potentiel étant les suivantes:

   a) une différence de potentiel V1 entre la surface du sol et la structure enterrée (2),

b) une différence de potentiel V2 entre la surface du sol et la structure enterrée (2), lorsque le potentiel de ladite structure est ramené au moyen d'une liaison électrique (9) à une électrode métallique (5), reposant sur le sol,

c) et au moins une troisième différence de potentiel V3 entre la surface du sol et la structure enterrée, obtenue en modifiant la différence de potentiel qui est engendrée par l'action de l'échange électrique existant entre ladite structure et le sol;

B.- la visualisation et l'enregistrement par un système de visualisation et d'acquisition de données de ces différences de potentiel;

C.- le traitement de ces données à l'aide d'un système de traitement de données pour calculer la différence (V1 - V2), et au moins la différence (V3 - V1).

D. - la détermination à partir de ces différences de l'effet corrosif du sol et de l'environnement sur la structure enterrée.

2. Procédé selon la revendication 1, caractérisé en ce que l'électrode (4) de potentiel de référence connu est une électrode du type $Cu/CuSO_4$.

3. Procédé selon l'une des revendications 1 et 2, caractérisé en ce que, pour mesurer la différence du potentiel V1, on dispose l'électrode (4) de potentiel de référence à la verticale du point analysé de ladite structure enterrée.

4. Procédé selon l'une des revendications 1 à 3, caractérisé en ce que, pour mesurer V2, une électrode $Cu/CuSO_4$ et une électrode métallique (41, 42) accolées sont placées à une distance $d_2$ au sol d'un même côté de la verticale du point analysé de ladite structure, $d_2$ étant comprise entre 0 et 50 cm.

5. Procédé selon l'une des revendications 1 à 4, caractérisé en ce que, pour modifier la différence de potentiel engendrée par l'échange électrique existant entre ladite structure et le sol, au moins un courant électrique est généré entre l'électrode $Cu/CuSO_4$ et l'électrode "structure enterrée" avant de mesurer V3.

6. Procédé selon l'une des revendications 1 à 4, caractérisé en ce que, pour modifier la différence de potentiel engendrée par l'échange électrique entre ladite structure et le sol, une électrode $Cu/CuSO_4$ - (43) est placée à une distance $d_3$ au sol de la verticale du point analysé de ladite structure, $d_3$ étant comprise entre 50 cm et 2 m.

7. Dispositif de mise en oeuvre du procédé selon l'une des revendications 1 à 6, caractérisé en ce qu'il comprend:

- un système de mesure (20) constitué d'un capteur comprenant au moins une électrode (21) $Cu/CuSO_4$ à laquelle est accolée une électrode métallique (22) contenant du fer, et d'au moins un voltmètre;

- au moins un système de visualisation et d'acquisition de données constitué d'au moins un amplificateur, d'au moins un convertisseur analogique numérique, d'au moins un enregistreur magnétique et d'au moins un appareil appartenant au groupe constitué par les afficheurs numériques et les enregistreurs électromécaniques,

- et un système de traitement des ces données, constitué de moyens de calcul informatiques.

8. Dispositif selon la revendication 7, caractérisé en ce que le dispositif de mesure comprend également au moins un générateur de courant (79).

9. Dispositif selon l'une des revendication 7 et 8, caractérisé en ce que le système de mesure est constitué d'un capteur à développement linéaire comprenant au moins deux électrodes $Cu/CuSO_4$ (21) en forme de roue creuse, X1 et X2, dont la périphérie correspond à la partie sensible de l'électrode, obtenue à partir de matériaux choisis dans le groupe constitué par le liège, le bois et toute matière poreuse inerte électriquement, dont les parois en forme de disque sont en une matière électriquement inerte, non poreuse, et qui contient un disque de cuivre (28) de diamètre inférieur à celui de ladite roue, et une solution saturée de sulfate de cuivre ($CuSO_4$), ces électrodes étant disposées en parallèle et maintenues solidaires, X2 à une distance $d_2$ de X1, et au moins une troisième électrode $Cu/CuSO_4$, X3, placée à une distance $d_3$ de X1, ledit capteur comprenant en outre au moins une électrode métallique (22), de préférence en acier doux, composée d'au moins un disque, accolé à au moins une des électrodes Cu/CuSO₄.

10. Dispositif selon l'une des revendications 7 et 8, caractérisé en ce que le système de mesure est constitué d'un capteur (1) à développement ponctuel comprenant au moins une électrode Cu/CuSO₄ - (4) obtenue à partir d'un cylindre en matière inerte électriquement, non poreuse, bouché à sa base par un bouchon (14) de matière poreuse, choisi dans le groupe constitué par le bois, le liège ou toute autre matière poreuse inerte électriquement, ledit cylindre contenant une solution (15) saturée de sulfate de cuivre, dans laquelle plonge une tige de cuivre (16) assurant la liaison électrique de ladite électrode, et d'une électrode métallique (5) enveloppant l'électrode Cu/CuSO₄ d'une gaine métallique de préférence en acier doux.

11. Dispositif selon l'une des revendications 6 à 9, caractérisé en ce que le traitement des données par des moyens de calcul informatiques consiste à normaliser les valeurs V1, V2 et V3, en fonction des potentiels réels de la ou des électrodes Cu/CuSO₄ utilisées, et a calculer la différence (V1 - V2), la différence (V3 - V1) et toutes autres différences de potentiel par rapport à V1.

12. Application du procédé d'analyse de l'effet corrosif selon l'une des revendications 1 à 6, ou du dispositif selon l'une des revendications 7 à 11, à la localisation sur le site des zones anodiques favorables à la corrosion, caractérisée par les opérations suivantes:
    - déplacer de façon continue ou discontinue le capteur du système de mesure le long de la structure enterrée sur le sol préalablement humidifié, pour assurer le contact capteur sol,
    - lire sur l'appareil de visualisation du système de visualisation et d'acquisition de données les valeurs de V1 et de V2,
    - et localiser et signaler les zones anodiques lorsque V1 est supérieur en valeur absolue à la valeur absolue de V2.

**Claims**

1. Continuous or discontinuous method for analysing the corrosive effect of the soil and of the environment on a buried metallic structure (2), characterised by the following operations:
   A. The measurement of at least two potential differences using at least one electrode (4) of known reference potential placed in contact with the surface of the soil, the said soil constituting the electrolyte and the buried structure (4) a metallic electrode, the potential differences being the following:
   (a) a potential difference V1 between the surface of the soil and the buried structure (2),
   (b) a potential difference V2 between the surface of the soil and the buried structure (2), when the potential of the said structure is taken back by means of an electrical connection (9) to a metallic electrode (5) resting on the soil,
   (c) and at least a third potential difference V3 between the surface of the soil and the buried structure, obtained by modifying the potential difference which is created by the action of the electric exchange existing between the said structure and the soil;
   B. The display and recording, by a display and data acquisition system, of these potential differences;
   C. The processing of these data using a data processing system in order to calculate the difference (V1 - V2) and at least the difference (V3 - V1);
   D. The determination from these potential differences of the corrosive effect of the soil and of the environment on the buried structure.

2. Method according to Claim 1, characterised in that the electrode (4) of known reference potential is an electrode of the Cu/CuSO₄ type.

3. Method according to one of Claims 1 and 2, characterised in that, in order to measure the potential difference V1, the electrode (4) of reference potential is placed vertically above the analysed point of the said buried structure.

4. Method according to one of Claims 1 to 3, characterised in that, in order to measure V2, a Cu/CuSO₄ electrode and a metallic electrode (41, 42) coupled together are placed on the soil at a distance $d_2$ on the same side of the vertical through the analysed point of the said structure, $d_2$ lying between 0 and 50 cm.

5. Method according to one of Claims 1 to 4, characterised in that, in order to modify the potential difference created by the electric exchange existing between the said structure and the soil, at least one electric current is generated between the Cu/CuSO$_4$ electrode and the "buried structure" electrode before measuring V3.

6. Method according to one of Claims 1 to 4, characterised in that, in order to modify the potential difference created by the electric exchange between the said structure and the soil, a Cu/CuSO$_4$ electrode (43) is placed on the soil at a distance d$_3$ from the vertical through the analysed point of the said structure, d$_3$ lying between 50 cm and 2 m.

7. Device for implementing the method according to one of Claims 1 to 6, characterised in that it comprises:
    - a measuring system (20) consisting of a sensor comprising at least one Cu/CuSO$_4$ electrode (21) to which is coupled a metallic electrode (22) containing iron, and at least one voltmeter;
    - at least one display and data acquisition system consisting of at least one amplifier, at least one analogue-to-digital converter, at least one magnetic recorder and at least one piece of equipment belonging to the group formed by digital display units and electromechanical recorders,
    - and a system for processing these data consisting of a data processing computer facility.

8. Device according to Claim 7, characterised in that the measuring device also comprises at least one current generator (79).

9. Device according to one of Claims 7 and 8, characterised in that the measuring system consists of a sensor for linear deployment comprising at least two Cu/CuSO$_4$ electrodes (21) in the shape of a hollow wheel, X1 and X2, the periphery of which corresponds to the sensitive part of the electrode, obtained from materials chosen from the group formed by cork, wood and any electrically inert porous substance, whose walls in the form of a disc are made of a non-porous electrically inert substance and which contains a copper disc (28) of diameter less than that of the said wheel, and a saturated solution of copper sulphate (CuSO$_4$), these electrodes being placed in parallel and held fixed to each other, X2 at a distance d$_2$ from X1, and at least a third Cu/CuSO$_4$ electrode, X3, placed at a distance d$_3$ from X1, the said sensor also comprising at least one metallic electrode (22), preferably made of mild steel, consisting of at least one disc, coupled to at least one of the Cu/CuSO$_4$ electrodes.

10. Device according to one of Claims 7 and 8, characterised in that the measuring system consists of a sensor (1) for point-by-point deployment comprising at least one Cu/CuSO$_4$ electrode (4) obtained from a cylinder made of a non-porous electrically inert substance blocked at its base by a plug (14) made of a porous material chosen from among the group formed by wood, cork or any other electrically inert porous substance, the said cylinder containing a saturated copper sulphate solution (15) in which is immersed a copper rod (16) providing for the electrical connection of the said electrode, and a metallic electrode (5) surrounding the Cu/CuSO$_4$ electrode with a metallic jacket preferably made of mild steel.

11. Device according to one of Claims 6 to 9, characterised in that the processing of data by the data processing computer facility involves normalising the values V1, V2, V3 in terms of the real potentials of the Cu/CuSO$_4$ electrode or electrodes used, and calculating the difference (V1 - V2), the difference (V3 - V1) and any other potential differences with respect to V1.

12. Application of the method for analysing the corrosive effect according to one of Claims 1 to 6, or of the device according to one of Claims 7 to 11, to the on-site location of the anodic regions likely to favour corrosion, characterised by the following operations:
    - continuously or discontinuously moving the sensor of the measuring system along the buried structure over the soil, which has been previously dampened in order to ensure contact between sensor and soil,
    - reading the values of V1 and V2 on the display unit of the display and data acquisition system,
    - and locating and indicating the anodic regions when V1 is greater in absolute value than the absolute value of V2.

10

**Patentansprüche**

1. Verfahren zur kontinuierlichen oder diskontinuierlichen Analyse der Korrosionswirkung des Erdbodens und der Umgebung auf eine eingegrabene Metallstruktur (2), **gekennzeichnet** durch die folgenden Schritte:

   A) Messen von mindestens zwei Potentialdifferenzen mit Hilfe mindestens einer mit der Oberfläche des Erdbodens in Berührung gebrachten Bezugspotentialelektrode (4), wobei der Erdboden den Elektrolyten und die eingegrabene Struktur (2) eine Metallelektrode bilden und es sich um die folgenden Potentialdifferenzen handelt:

   a) eine Potentialdifferenz V1 zwischen der Erdbodenoberfläche und der eingegrabenen Struktur (2),

   b) eine Potentialdifferenz V2 zwischen der Erdbodenoberfläche und der eingegrabenen Struktur (2), wenn das Potential der eingegrabenen Struktur (2) mittels einer elektrischen Verbindung (9) zu einer auf dem Erdboden ruhenden Metallelektrode (5) rückgeführt wird, und

   c) mindestens eine dritte Potentialdifferenz V3 zwischen der Erdbodenoberfläche und der eingegrabenen Struktur (2), welche durch Verändern derjenigen Potentialdifferenz erhalten wird, die durch den zwischen der eingegrabenen Struktur (2) und dem Erdboden bestehenden elektrischen Austausch bewirkt wird;

   B) Anzeigen und Speichern dieser Potentialdifferenzen durch ein Datenanzeige- und -sammelsystem;

   C) Verarbeiten dieser Daten mit Hilfe eines Datenverarbeitungssystems, um die Differenz (V1 - V2) und mindestens die Differenz (V3 - V1) zu berechnen;

   D) Bestimmen der Korrosionswirkung des Erdbodens und der Umgebung auf die eingegrabene Struktur (2) aus diesen Differenzen.

2. Verfahren nach Anspruch 1, dadurch **gekennzeichnet,** daß es sich bei der Bezugspotentialelektrode (4) um eine $Cu/CuSO_4$-Elektrode handelt.

3. Verfahren nach Anspruch 1 oder 2, dadurch **gekennzeichnet,** daß die Bezugspotentialelektrode (4) vertikal über dem analysierten Punkt der eingegrabenen Struktur (2) positioniert wird, um die Potentialdifferenz V1 zu messen.

4. Verfahren nach Anspruch 1, 2 oder 3, dadurch **gekennzeichnet,** daß eine $Cu/CuSO_4$-Elektrode und eine Metallelektrode zusammengefügt und gemeinsam (42) in einem Abstand $d_2$ zwischen 0 und 50 cm von der Vertikalen des analysierten Punktes der eingegrabenen Struktur positioniert werden, um die Potentialdifferenz V2 zu messen.

5. Verfahren nach einem der Ansprüche 1 bis 4, dadurch **gekennzeichnet,** daß mindestens ein elektrischer Strom zwischen der $Cu/CuSO_4$-Elektrode und der von der eingegrabenen Struktur gebildeten Elektrode vor dem Messen der Potentialdifferenz V3 erzeugt wird, um diejenige Potentialdifferenz zu verändern, welche durch den zwischen der eingegrabenen Struktur und dem Erdboden bestehenden elektrischen Austausch bewirkt wird.

6. Verfahren nach einem der Ansprüche 1 bis 4, dadurch **gekennzeichnet,** daß eine $Cu/CuSO_4$-Elektrode (43) in einem Abstand $d_3$ zwischen 50 cm und 2 m von der Vertikalen des analysierten Punktes der eingegrabenen Struktur positioniert wird, um diejenige Potentialdifferenz zu verändern, welche durch den zwischen der eingegrabenen Struktur und dem Erdboden bestehenden elektrischen Austausch bewirkt wird.

7. Vorrichtung zur Durchführung des Verfahrens nach einem der Ansprüche 1 bis 6, **gekennzeichnet** durch

   ein Meßsystem (20) bestehend aus einem Meßfühler mit mindestens einer $Cu/CuSO_4$-Elektrode (21), welcher eine eisenhaltige Metallelektrode (22) hinzugefügt ist, und mindestens einem Voltmeter,

   mindestens ein Datenanzeige und -sammelsystem bestehend aus mindestens einem Verstärker, mindestens einem Analog/Digital-Umsetzer, mindestens einem Magnetspeicher und mindestens einem Gerät aus der Gruppe der digitalen Anzeigevorrichtungen sowie elektromechanischen Speicher und

   ein System zur Verarbeiung dieser Daten, welches aus Rechenmitteln der Informatik besteht.

**8.** Vorrichtung nach Anspruch 7, dadurch **gekennzeichnet,** daß das Meßsystem außerdem mindestens einen Stromgenerator (79) aufweist.

**9.** Vorrichtung nach Anspruch 7 oder 8, dadurch **gekennzeichnet,** daß das Meßsystem aus einem linear erfassenden Meßfühler mit

mindestens zwei $Cu/CuSO_4$-Elektroden X1 und X2, welche parallel zueinander in einem gegenseitigen Abstand $d_2$ angeordnet und jeweils als Hohlrad (21) ausgebildet sind, an dessen Umfang der empfindliche Elektrodenteil (27) aus Kork und/oder Holz und/oder einem anderen porösen sowie elektrisch inerten Material angeordnet ist, dessen scheibenförmige Seitenwände (26) aus einem elektrisch inerten Material ohne Poren bestehen und das eine Kupferscheibe (28) mit einem Durchmesser kleiner als der Raddurchmesser sowie eine gesättigte Kupfersulfatlösung (29) enthält,

mindestens einer dritten $Cu/CuSO_4$-Elektrode X3, welche in einem Abstand $d_3$ von der $Cu/CuSO_4$-Elektrode X1 angeordnet ist, und

mindestens einer Metallelektrode (22), vorzugsweise aus weichem Stahl, welche von mindestens einer zu mindestens einer der $Cu/CuSO_4$-Elektroden hinzugefügten Scheibe gebildet ist,

besteht.

**10.** Vorrichtung nach Anspruch 7 oder 8, dadurch **gekennzeichnet,** daß das Meßsystem aus einem punktweise erfassenden Meßfühler (1) mit

mindestens einer $Cu/CuSO_4$-Elektrode (4), welche einen Zylinder (13) aus einem elektrisch inerten Material ohne Poren aufweist, der an der Basis durch einen Stopfen (14) aus Kork und/oder Holz und/oder einem anderen porösen sowie elektrisch inerten Material verschlossen ist und eine gesättigte Kupfersulfatlösung (15) enthält, in die eine den elektrischen Anschluß der Elektrode ermöglichende Kupferstange (16) taucht, und

einer Metallelektrode (5), welche die $Cu/CuSO_4$-Elektrode als Metallhülse, vorzugsweise aus weichem Stahl, umhüllt,

besteht.

**11.** Vorrichtung nach Anspruch 7, 8, 9 oder 10, dadurch **gekennzeichnet,** daß die Datenverarbeitung durch die Rechenmittel der Informatik darin besteht, die Werte V1, V2 und V3 in Abhängigkeit von dem oder den tatsächlichen Potentialen der verwendeten $Cu/CuSO_4$-Elektrode oder $Cu/CuSO_4$-Elektroden zu normalisieren und die Differenz (V1 - V2), die Differenz (V3 - V1) sowie alle anderen Potentialdifferenzen bezüglich des Wertes V1 zu berechnen.

**12.** Anwendung des Verfahrens nach einem der Ansprüche 1 bis 6 oder der Vorrichtung nach einem der Ansprüche 7 bis 11 zur Lokalisierung der korrosionsanfälligen anodischen Zonen am Einsatzort, **gekennzeichnet** durch die folgenden Schritte:

kontinuierliches oder diskontinuierliches Bewegen des Meßfühlers des Meßsystems auf dem zur Gewährleistung des Meßfühler/Erdboden-Kontaktes zuvor angefeuchteten Erdboden an der eingegrabenen Struktur entlang,

Ablesen der Werte der Potentialdifferenzen V1 und V2 vom Anzeigegerät des Datenanzeige- und -sammelsystems und

Lokalisieren sowie Signalisieren der anodischen Zonen, wenn der absolute Wert der Potentialdifferenz V1 größer als der absolute Wert der Potentialdifferenz V2 ist.

FIG.1

FIG.2A

FIG.2B

14

FIG.3

FIG.4A

FIG.4B

FIG.5

EP 0 229 573 B1

FIG.6